# EUROPEAN PATENT SPECIFICATION

(11) **EP 3 851 099 B1**
(45) Date of publication and mention of the grant of the patent: **31.12.2025**
(21) Application number: 19860298.9
(22) Date of filing: 11.09.2019
(51) Int. Cl.: A61K 31/165, A61K 31/167, A61K 31/341, A61P 1/16, A61P 11/00, A23L 33/10, A23K 20/111, A23K 20/121

(54) **COMPOSITION FOR TREATING FIBROTIC DISEASES, COMPRISING BENZHYDRYL THIOACETAMIDE COMPOUND AS ACTIVE INGREDIENT**
ZUSAMMENSETZUNG ZUR BEHANDLUNG VON FIBROTISCHEN ERKRANKUNGEN, MIT BENZHYDRYL-THIOACETAMID-VERBINDUNG ALS WIRKSTOFF
COMPOSITION DESTINÉE À TRAITER DES MALADIES FIBROTIQUES, COMPRENANT UN COMPOSÉ BENZHYDRYL THIOACÉTAMIDE EN TANT QUE PRINCIPE ACTIF

(30) Priority: 14.09.2018 KR 20180110442
(43) Date of publication of application: 21.07.2021
(73) Proprietor: Cellionbiomed Inc., Sejong 30033 (KR)
(72) Inventor: SUH, Suk-Hyo, Goyang-si, Gyeonggi-do 10323 (KR); KIM, Seong-Jin, Sejong 30062 (KR)
(74) Representative: Marks & Clerk LLP
(86) International application number: PCT/KR2019/011834
(87) International publication number: WO 2020/055166

(56) References cited:
- WO-A1-2016/038239
- KR-A- 20130 044 930
- KR-A- 20130 080 364
- US-A1- 2014 296 333
- GRGIC IVICA ET AL: "Renal fibrosis is attenuated by targeted disruption of K Ca 3.1 potassium channels", vol. 106, no. 34, 25 August 2009 (2009-08-25), pages 14518 - 14523, XP093018131, ISSN: 0027-8424, Retrieved from the Internet <URL:https://www.ncbi.nlm.nih.gov/pmc/articles/PMC2726862/pdf/zpq14518.pdf> DOI: 10.1073/pnas.0903458106
- ROACH KATY MORGAN ET AL: "The K+ Channel KCa3.1 as a Novel Target for Idiopathic Pulmonary Fibrosis", vol. 8, no. 12, 1 January 2013 (2013-01-01), pages 1 - 18, XP093018146, Retrieved from the Internet <URL:https://www.ncbi.nlm.nih.gov/pmc/articles/PMC3877378/pdf/pone.0085244.pdf> DOI: 10.1371/journal.pone.0085244
- SHINKYU CHOI ET AL: "Modafinil inhibits K3.1 currents and muscle contraction via a cAMP-dependent mechanism", PHARMACOLOGICAL RESEARCH, ELSEVIER, AMSTERDAM, NL, vol. 66, no. 1, 24 February 2012 (2012-02-24), pages 51 - 59, XP028422732, ISSN: 1043-6618, [retrieved on 20120305], DOI: 10.1016/J.PHRS.2012.02.009
- CHOI SHINKYU ET AL: "Anti-inflammatory and anti-fibrotic effects of modafinil in nonalcoholic liver disease", BIOMEDICINE & PHARMACOTHERAPY, ELSEVIER, FR, vol. 144, 28 October 2021 (2021-10-28), XP086861406, ISSN: 0753-3322, [retrieved on 20211028], DOI: 10.1016/J.BIOPHA.2021.112372
- PAKA LATHA ET AL: "Anti-steatotic and anti-fibrotic effects of the KCa3.1 channel inhibitor, Senicapoc, in non-alcoholic liver disease", WORLD JOURNAL OF GASTROENTEROLOGY, vol. 23, no. 23, 21 June 2017 (2017-06-21), CN, pages 4181, XP093136080, ISSN: 1007-9327, Retrieved from the Internet <URL:https://www.ncbi.nlm.nih.gov/pmc/articles/PMC5483492/pdf/WJG-23-4181.pdf> DOI: 10.3748/wjg.v23.i23.4181
- SHEPHERD M. C. ET AL: "S139 The K+ channel KCa3.1 is a novel target for the treatment of idiopathic pulmonary fibrosis", THORAX, vol. 65, no. Suppl 4, 1 December 2010 (2010-12-01), GB, pages A63 - A63, XP093135920, ISSN: 0040-6376, Retrieved from the Internet <URL:https://thorax.bmj.com/content/thoraxjnl/65/Suppl_4/A63.1.full.pdf> DOI: 10.1136/thx.2010.150946.40
- WULFF HEIKE ET AL: "Design of a potent and selective inhibitor of the intermediate-conductance Ca 2? -activated K ? channel, IKCa1: A potential immunosuppressant", PROC. NATL. ACAD. SCI. USA, 1 July 2000 (2000-07-01), pages 8151 - 8156, XP093292026, Retrieved from the Internet <URL:https://pmc.ncbi.nlm.nih.gov/articles/PMC16685/pdf/pq008151.pdf>
- STOCKER JONATHAN W. ET AL: "ICA-17043, a novel Gardos channel blocker, prevents sickled red blood cell dehydration in vitro and in vivo in SAD mice", BLOOD, vol. 101, no. 6, 15 March 2003 (2003-03-15), AMSTERDAM, NL, pages 2412 - 2418, XP093292056, ISSN: 0006-4971, Retrieved from the Internet <URL:https://pubmed.ncbi.nlm.nih.gov/12433690/> DOI: 10.1182/blood-2002-05-1433
- CRANE G. J. ET AL: "Small- and Intermediate-Conductance Calcium-Activated K + Channels Provide Different Facets of Endothelium-Dependent Hyperpolarization in Rat Mesenteric Artery", THE JOURNAL OF PHYSIOLOGY, vol. 553, no. 1, 1 November 2003 (2003-11-01), GB, pages 183 - 189, XP093292057, ISSN: 0022-3751, Retrieved from the Internet <URL:https://pmc.ncbi.nlm.nih.gov/articles/PMC2343487/pdf/tjp0553-0183.pdf> DOI: 10.1113/jphysiol.2003.051896
- K�HLER R. ET AL: "The endothelium-derived hyperpolarizing factor: insights from genetic animal models", KIDNEY INTERNATIONAL, vol. 72, no. 2, 1 July 2007 (2007-07-01), United States, pages 145 - 150, XP093292060, ISSN: 0085-2538, Retrieved from the Internet <URL:https://www.sciencedirect.com/science/article/pii/S0085253815526197?via%3Dihub> DOI: 10.1038/sj.ki.5002303
- HAN, J.: "Modafinil attenuates inflammation via inhibiting Akt/NF-KB pathway in apoE-deficient mouse model of atherosclerosis", INFLAMMOPHARMACOLOGY, vol. 26, no. 2, 21 August 2017 (2017-08-21), pages 385 - 393, XP036461873, DOI: 10.1007/s10787-017-0387-3
- JUNG, J.-C.: "Simple synthesis of modafinil derivatives and their anti-inflammatory activity", MOLECULES, vol. 17, 2012, pages 10446 - 10458, XP055693169
- GAN, S. I. ET AL.: "Modafinil in the Treatment of Debilitating Fatigue in Primary Biliary Cirrhosis: A Clinical Experience", DIG DIS SCI, vol. 54, 2009, pages 2242 - 2246, XP019728733

## Description

### [Technical Field]

The present disclosure relates to a composition for treating a fibrotic disease, which includes a benzhydryl thioacetamide compound as an active ingredient, and more particularly, to a composition for treating a fibrotic disease, which suppresses the expression of K_{Ca}2.3 channel proteins in a cell membrane, and has an excellent effect of treating, particularly, liver fibrosis and pulmonary fibrosis. The invention relates to a composition for use in treating liver fibrosis or pulmonary fibrosis, comprising a benzhydryl thioacetamide compound as defined in the claims or a pharmaceutically acceptable salt thereof as an active ingredient.

### [Background Art]

Fibrosis is a phenomenon of excessively accumulating an extracellular matrix such as collagen in tissue, and occurs during the process of tissue damage and recovery. The fibrosis may occur in all organs in the body, and it easily occurs, particularly, when an injury is severe and extensive and when the process of tissue injury and recovery is repeated as in chronic diseases. When fibrosis occurs, damaged tissue is replaced with fibrous tissue, reducing the functions of an organ. Therefore, when fibrosis occurs extensively, the organ function is greatly reduced, thereby causing various types of diseases. Particularly, when fibrosis occurs in the internal organs that directly affect life, such as the liver, lung, kidney and heart, it may have a fatal effect on health.

Generally, a process of fibrosis may include 1) the exposure to a fibrosis-inducing diseases(normally, chronic diseases) or materials, and 2) the resulting fibrotic process (inflammation, fibrosis, and angiogenesis). When inflammation and injury occur due to a fibrosis-inducing disease or material, fibrosis and angiogenesis are accelerated by growth factors and cytokines, which are secreted in cells participating in this process. Therefore, fibrotic diseases may be treated by removing fibrosis causes (diseases or materials) or suppressing the fibrotic process.

However, it is virtually impossible to completely remove the causes of fibrosis. The causes are unknown in many fibrotic diseases such as idiopathic pulmonary fibrosis. Even if the causes of fibrotic diseases, such as chronic viral hepatitis, steatohepatitis, diabetes causing heart or kidney fibrosis, and aging frequently causing various types of fibrotic diseases, are known, it is often impossible to cure the cause diseases completely. Therefore, treatment of fibrotic diseases requires concurrent treatment for inhibiting the fibrotic process (inflammation, fibrosis, angiogenesis) as well as treatment of a causative disease. However, no therapeutic agent for inhibiting the fibrotic process has been developed.

In the fibrotic process, the formation of myofibroblasts and the activation of hepatic stellate cells (in the liver, the activated hepatic stellate cells serve as myofibroblasts) are very important. The formation of myofibroblasts including the activation of hepatic stellate cells is induced by activation of fibroblasts or smooth muscle cells or endothelial-mesenchymal transition of endothelial cells. In addition, when myofibroblasts are formed, the number of the myofibroblasts greatly increases due to active cell proliferation, the production of an extracellular matrix such as collagen increases, and angiogenesis is stimulated due to active vascular endothelial cell proliferation. Such a fibrotic process, that is, myofibroblast formation (including the activation of hepatic stellate cells), myofibroblast proliferation, extracellular matrix production, the activation of vascular endothelial cells and angiogenesis occur via intracellular Ca²⁺-dependent signaling pathways. Therefore, Ca²⁺ plays a very important role in the fibrotic process.

For the increase in Ca²⁺ in fibroblasts, hepatic stellate cells and vascular endothelial cells, Ca²⁺-activated K⁺ channels, that is, "K_{Ca} channels" are significantly important. The K⁺ channel activation-induced hyperpolarization promote Ca²⁺ influx through Ca²⁺ entry channels in these cells. The K_{Ca} channels playing such a role in these cells are the K_{Ca}2.3 channel and the K_{Ca}3.1 channel. These two K⁺ channels are similar in structure and function, but there is a difference in cells in which these channels are distributed.

Since mRNA is found in most tissue cells, the K_{Ca}2.3 channel is possibly distributed in most tissues in the body (Naunyn Schmiedebergs Arch Pharmacol .2004; 369(6):602-15), and widely distributed in the liver, nerves and vascular endothelial cells. On the other hand, the K_{Ca}3.1 channel is generally distributed in vascular endothelial cells, fibroblasts, immune cells and red blood cells (Curr Med Chem. 2007;14(13):1437-57; Expert Opin Ther Targets. 2013;17(10):1203-1220).

As described above, K_{Ca}2.3 or K_{Ca}3.1 channels, which are considered to significantly contribute to the progression of fibrosis via promoting Ca2+ influx through Ca²⁺ entry channels, are being studied as the main targets of therapeutic agents for fibrotic diseases. Particularly, it has been reported that a selective inhibitor of the K_{Ca}2.3 channel, apamin, has an inhibitory effect on endothelial-mesenchymal transition that is critical for the fibrotic process, and has a therapeutic effect on liver fibrosis and biliary fibrosis (Biochem Biophys Res Commun. 2014; 450(1): 195- 201; Int J. Mol Med. 2017;39(5):1188-1194).

The ion channel inhibitors, that have been developed so far, inhibits cell functions via inhibiting the activity of an ion channel (inhibiting the flow of ions through a channel protein). Since the number of channel proteins expressed in a cell membrane affect cell function, cell functions can also be regulated by reducing the number of channel proteins expressed in a cell membrane (inhibition of the expression of a channel protein in a cell membrane). No drug for regulating an expression level of a channel protein in a cell membrane has been developed so far, and molecules to regulate the expression level can be a new therapeutic for various diseases (Chem Med Chem. 2012;7(10):1741-1755). Particularly, since the expression of the K_{Ca}2.3 channel is increased by growth factors in fibrotic diseases, drugs for inhibiting the expression of K_{Ca}2.3 channel proteins may be developed as therapeutic agents for fibrotic diseases.

Meanwhile, in U.S. Patent Nos. 4,066,686 and US 4,177,290, a benzhydryl sulfinyl acetamide derivative included in the present invention is suggested as drugs for treating central nervous system disorders, and this compound was developed as a medication to treat narcolepsy by Lafon, France, and is sold under the generic name "modafinil." Adrafinil, which is known as the modafinil precursor, that is, diphenylmethyl-thioacetohydroxamic acid, was also developed as a medication having the same efficacy as modafinil (CNS Drug Reviews Vol5, No.3 193-212, 1999).

In addition, according to U.S. Patent No. 4,927,855, it has been suggested that the R-isomer of modafinil (Lafon), that is, (-)-benzhydryl sulfinyl acetamide, has therapeutic effects on anti-depressant, hypersomnia and Alzheimer's disease, according to US Patent No. 6,180,678, it has been suggested that R-modafinil (Vetoquinol, France) is effective in treatment of behavioral problems of an older dog, improvement in learning effect, bladder control, and memory improvement, and according to US Patent No. 9,637,447, it has been suggested that 2-[bis(4-fluorophenyl)methanesulfinyl]acetamide, known under the generic name "lauflumide," is effective against attention-deficit hyperactivity disorder (ADHD), narcolepsy, epilepsy, and lethargy.

In addition, the inventors have reported in Korean Patent Nos. 10-1345860 and 10-1414831 and the corresponding U.S. Patent No. 9,259,412 that modafinil and their derivatives can be used as drugs to treat vascular diseases and K_{Ca}3.1 channel-mediated diseases, that is, cancer and autoimmune diseases by increasing cAMP to relax blood vessels, and inhibit K_{Ca}3.1 current.

Roach et al. disclose in Plos One, 8(12), 2013, 1-18, that blocking KCa3.1 may offer a novel approach to treating idiopathic pulmonary fibrosis (IPF).

Paka et al. disclose in World J. Gastroenterology, 23(23), 2017, 4181, that the KCa3.1 inhibitor Senicapoc has antifibrotic effect in non-alcoholic liver disease.

Shepherd et al. disclose in Thorax, 65, Suppl. 4, 2010, A63 that KCa3.1 expression is increased in a model of pulmonary fibrosis and inhibition with the KCa3.1 inhibitor TRAM-34 significantly improves pathological outcome.

### [Disclosure]

### [Technical Problem]

In the process of studying the pharmaceutical activity of benzhydryl thioacetamide compounds including benzhydryl sulfinyl acetamide derivatives, the inventors found that such compounds surprisingly suppress the expression of the K_{Ca}2.3 channel in a cell membrane, and further have a therapeutic effect on fibrotic diseases in mouse models.

The present disclosure is directed to providing a novel composition for treating fibrotic diseases, which includes a benzhydryl thioacetamide compound or a pharmaceutically acceptable salt thereof as an active ingredient. For reference, the "benzhydryl thioacetamide compound" used herein is used as a concept including "benzhydryl sulfinyl acetamide compound."

### [Technical Solution]

A composition for treating a fibrotic disease according to the present disclosure includes a benzhydryl thioacetamide compound represented by Formula A below or a pharmaceutically acceptable salt thereof as an active ingredient. 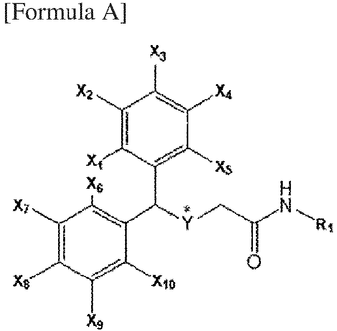 [In Formula A, X₁~X₁₀ may each be independently hydrogen (H) or fluorine (F), all of which may be the same as or different from each other; Y is sulfur (S) or sulfoxide (S=O), * indicates a chiral position; R₁ is any one of hydrogen, a methyl group, an ethyl group, a methoxy group, an ethoxy group, a hydroxyl group, and a carbon compound having 3 to 6 carbon atoms.]

In the compound of Formula A, X₁~X₁₀ are each independently hydrogen (H) or fluorine (F), Y is sulfur (S), and R₁ is hydrogen (H).

In the compound of Formula A, X₁~X₁₀ are each independently hydrogen (H) or fluorine (F), Y is sulfoxide (S=O), and R₁ is hydrogen (H).

The compound of Formula A has an effect of suppressing the expression of the KCa2.3 channel protein in a cell membrane.

The compound of Formula A has efficacy in treating, particularly, liver fibrosis and pulmonary fibrosis. The benzhydryl thioacetamide compounds according to the invention are as defined in the claims.

### [Advantageous Effects]

It was confirmed that the benzhydryl thioacetamide compound according to the present invention has an effect of suppressing the expression of a K_{Ca}2.3 channel protein in an *in vitro* experiment for culture cells, and further has an effect of inhibiting inflammation and fibrosis and improving liver functions in an *in vivo* experiment for mouse models in which liver and lung diseases are induced.

Accordingly, the benzhydryl thioacetamide compound according to the present invention can be effectively used as a pharmaceutical composition for treating various types of inflammatory and fibrotic diseases that occur in the human body, and particularly, inflammatory and fibrotic diseases in the liver and lungs, and is expected to be developed as a medication for animals, if needed.

### [Description of Drawings]

FIGS. 1A to 1C show effects of PDGF, TGF_{β}, and a compound of Formula A1 according to the present invention on the expression of K_{Ca}2.3 and K_{Ca}3.1 channels in vascular endothelial cells, fibroblasts, and hepatic stellate cells.
FIGS. 2A and 2B show the effects of a compound of Formula A1 on the expression of a fibrosis marker (FIG. 2A) and cell proliferation (FIG. 2B) in fibroblasts exposed to TGF_{β} inducing an increase in expression of a K_{Ca}2.3 channel and fibrosis.
FIG. 3 shows the effects of compounds of Formulas A1 to A9 according to the present invention on the expression of a K_{Ca}2.3 channel in hepatic stellate cells.
FIG. 4 shows the K_{Ca}2.3 current in hepatic stellate cells reduced in expression of a K_{Ca}2.3 channel due to exposure to compounds of Formulas A2 to A4 and A9 according to the present invention for 24 hours.
FIG. 5 shows the effects of compounds of Formulas A2 to A5, A8 and A9 according to the present invention on cell proliferation in fibroblasts exposed to TGF_{β} or PDGF inducing fibrosis for 24 hours.
FIGS. 6A to 6D show the inflammation inhibitory and fibrosis inhibitory effects of the compound of Formula A1 according to the present invention and isomers thereof in TAA-induced liver disease mouse models by a histological or immunohistochemical method.
FIGS. 7A and FIG. 7B show results of testing liver functions according to the presence or absence of the administration of the compounds of Formulas A1 to A5 according to the present invention in TAA or western diet-induced liver disease mouse models (FIG. 7A or 7B).
FIGS. 8A and 8B show the change in mRNA expression of inflammatory cytokines according to the administration of the compounds of Formulas A1, and A2 to A5 according to the present invention in TAA-induced liver disease mouse models, and FIG. 8C shows the change in mRNA expression of inflammatory cytokines according to the administration of the compound of Formula A1 in western diet (WD)-induced liver disease mouse models.
FIG. 9 shows the change in mRNA expression of fibrosis markers according to the presence or absence of the administration of the compound of Formula A1 in TAA-induced liver disease mouse models.
FIGS. 10A and 10B show the effects of the R-isomer and S-isomer of the compound of Formula A1 on the expression of inflammation marker (FIG. 10A) and fibrosis marker (FIG. 10B) proteins in TAA-induced liver disease mouse models.
FIG. 11 shows the effects of the R-isomer and S-isomer of the compound of Formula A1 on the expression of a K_{Ca}2.3 channel protein in TAA-induced liver disease mouse models.
FIG. 12 shows the effects of the compound of Formula A9 on pulmonary inflammation and fibrosis in bleomycin-induced pulmonary fibrosis mouse models.
FIGS. 13A and 13B show the effect of the compound of Formula A9 on the expression of inflammation marker (FIG. 13A) and fibrosis marker (FIG. 13B) proteins in bleomycin-induced pulmonary fibrosis mouse models.

### [Modes of the Invention]

A benzhydryl thioacetamide compound according to the present invention is selected from the group consisting of compounds of Formulas A1 to A9 below.

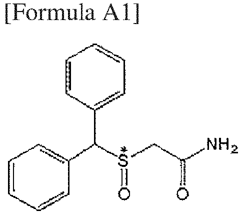

The compound of Formula A1 is known under the generic name "modafinil," and currently used as a medication to treat hypnolepsy, and clinical trials for use in treatment of other psychiatric diseases are ongoing. The chemical name of modafinil is 2-(benzhydrylsulfinyl)acetamide, and may be synthesized by a known method or commercially available.

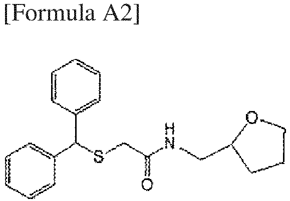

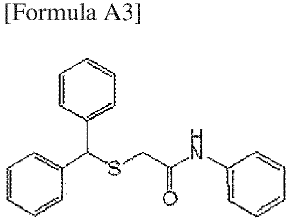

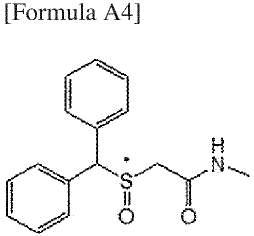

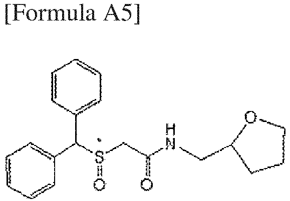

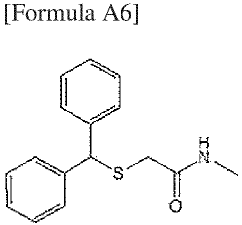

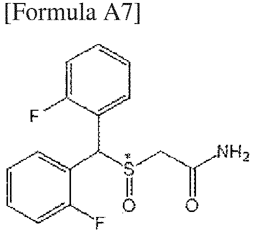

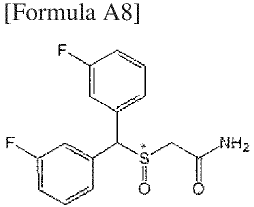

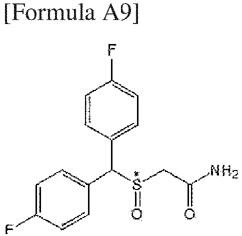

All of the compounds of Formulas A2 to A9 have the effect of suppressing the expression of a K_{Ca}2.3 channel protein in a cell membrane according to the same mechanism as the modafinil, and further have a therapeutic effect on fibrotic diseases in the human body. According to the invention, they are for use in treating liver fibrosis or pulmonary fibrosis.

Among these, the compound of Formula A9 is known under the generic name "lauflumide."

The chemical names of the compounds of Formulas A1 to A9 are as follows. The code names listed in parentheses at the end of each chemical name are code names used in the following examples by the inventors.
1) Formula A1; 2-(benzhydrylsulfinyl)acetamide (CBM-N1)
2) Formula A2; 2-(benzhydrylthio)-N-[(tetrahydrofuran-2-yl)methyl]acetamide (CBM-N2)
3) Formula A3; 2-(benzhydrylthio)-N-phenylacetamide (CBM-N3)
4) Formula A4; 2-(benzhydrylsulfinyl)-N-methylacetamide (CBM-N4)
5) Formula A5; 2-(benzhydrylsulfinyl)-N-[(tetrahydrofuran-2-yl)methyl]acetamide (CBM-N5)
6) Formula A6; 2-(benzhydrylthio)-ene-methylacetamide (CBM-N6)
7) Formula A7; 2-[bis(2-fluorophenyl)methanesulfinyl]acetamide (CBM-N7)
8) Formula A8; 2-[bis(3-fluorophenyl)methanesulfinyl]acetamide (CBM-N8)
9) Formula A9; 2-[bis(4-fluorophenyl)methanesulfinyl]acetamide (CBM-N9)

The compounds of Formulas A2 to A6 may be synthesized by the methods disclosed in Korean Patent No. 10-1345860, or commercially available, but no effective methods of preparing the compounds of Formulas A7 to A9 are known. Thus, in the present disclosure, methods of preparing the compounds of Formulas A7 to A9 were described as examples.

The pharmaceutical composition for use according to the present invention includes a pharmaceutically acceptable salt of the compound of Formula A1-A9. Here, the "pharmaceutically acceptable salt" may commonly include a metal salt, a salt with an organic base, a salt with an inorganic acid, a salt with an organic acid, or a salt with a basic or acidic amino acid. In addition, the pharmaceutical composition according to the present invention may include both of a solvate and a hydrate of the compound of Formula A1-A9, also include all of available stereoisomers, and further include a crystalline or amorphous form of each compound.

The pharmaceutical composition according to the present invention may be formulated in the form of a tablet, a pill, a powder, a granule, a capsule, a suspension, a liquid for internal use, an emulsion, a syrup, an aerosol, or a sterile injection solution according to a conventional method. In addition, the pharmaceutical composition of the present invention may be administered either orally or parenterally according to the purpose of use, and parenteral administration may be performed by dermal injection for external use, intraperitoneal injection, intrarectal injection, subcutaneous injection, intravenous injection, intramuscular injection or intracardiac injection.

A dose of the pharmaceutical composition according to the present invention may vary according to a patient's body weight, age, sex, health condition, diet, an administration duration, an administration method, an excretion rate, and the severity of a disease. A daily dose is preferably 0.2 to 20 mg/kg, and more preferably 0.5 to 10 mg/kg based on an active ingredient, and may be administered once or twice daily, but the present invention is not limited thereto.

### [Examples]

### 1) Synthesis of compounds

### 1-1) Synthesis of compound of Formula A9

A method of synthesizing a compound (lauflumide) of Formula A9 will be described with reference to the following reaction scheme. 24 g of 4,4'-bisdfluoro benzhydrol (I) was put into a 500 mL round-bottom flask, dissolved in 150 mL of added trifluoroacetic acid, and stirred with 12.05 g of added thigolic acid for approximately 2 hours, followed by confirmation of the termination of the reaction by thin-layer chromatography. The reaction product was subjected to vacuum distillation to remove the trifluoroacetic acid, neutralized and extracted with an ethyl acetate organic solvent. The resulting extract was dried with magnesium sulfate, thereby obtaining 34.8 g of compound (II), which is a sticky yellow oil, with a quantitative yield.

34.8 g of the compound (II) was dissolved in 250 mL of anhydrous ethanol, and 4.2 g of concentrated sulfuric acid was added, followed by reflux for 8 hours. Subsequently, the resulting product was cooled to room temperature, concentrated to remove ethanol, dissolved in a methylene chloride solvent, and washed with water twice. The resulting product was washed again with a 5% NaHCO₃ solution, and dried with anhydrous magnesium sulfate, thereby obtaining 39.1 g of compound (III), which is a yellow oil, with a quantitative yield.

34.3 g of the compound (III) was put into a round-bottom flask (500 mL), 210 mL of methanol was added, 21.4mL of an acid catalyst (the acid catalyst was prepared by dissolving 4g of sulfuric acid in 90 mL of isopropyl alcohol), and a 35% H₂O₂ solution was slowly added, followed by stirring overnight at room temperature. Subsequently, 70 g of sodium chloride (NaCl) was added, extracted with a methylene chloride solution three times, dried with anhydrous magnesium sulfate and concentrated, thereby obtaining compound (IV) with a quantitative yield.

5.1 g of the compound (IV) was added to a round-bottom flask (100 mL) with 13 mL of methanol, 1.3 g of ammonium chloride (NH₄Cl) was added, and 98 mL of a concentrated ammonium hydroxide solution (NH₄OH) was then added. After stirring overnight, a white emulsion-type solution was filtered, thereby obtaining 4 g of a solid powder. The 4 g of the solid powder was dissolved in 28 g of isopropyl alcohol, refluxed and cooled to a room temperature, thereby obtaining 2.1 g of 2-[bis(4-fluorophenyl)methanesulfinyl] acetamide, which is a white crystal compound, represented by Formula A9.
¹H NMR(DMSO-d6): δ 7.68 (bs, 1H); 7.56-7.51 (m, 4H), 7.33 (bs, 1H), 7.29-7.24 (m, 4H), 5.4 (s, 1H); 3.4 (d, J = 13.6 Hz, 1H); 3.16 (d, J =13.6 Hz, 1H)

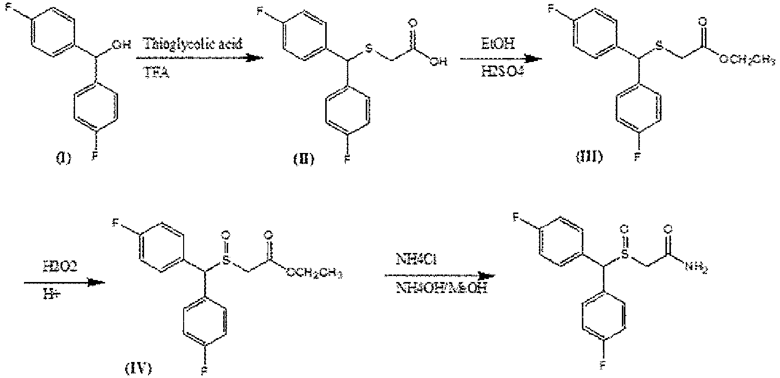

### 1-2) Synthesis of compound of Formula A8

3,3'-bisfluoro benzhydrol was synthesized by a conventional method (Tetrahedron Lett, vol 58, 442, 2017, EP 1,433,744, J. Med. Chem. vol 40, 851, 1997). This compound was used as a starting material, and a compound of Formula A8, that is, 2-[bis(3-fluorophenyl)methanesulfinyl]acetamide, was synthesized by the method of synthesizing the compound of Formula A9.
¹H NMR(DMSO-d6): δ 7.68 (bs, 1H); 7.5-7.2 (m, 9H), 5.4 (s, 1H); 3.4 (d, 1H); 3.16 (d, Hz, 1H)

### 1-3) Synthesis of compound of Formula A7

2,2'-bisfluoro benzhydrol was synthesized by a conventional method (EP 1,661,930, J. Med. Chem. vol 51, #4, 976, 2008). This compound was used as a starting material, and the compound of Formula A7, that is, 2-[bis(2-fluorophenyl)methanesulfinyl]acetamide. was synthesized using the method of synthesizing the compound of Formula A9.
¹H NMR(DMSO-d6): δ 7.68 (bs, 1H); 7.5-7.2 (m, 9H), 7.33 (bs, 1H), 5.4 (s, 1H); 3.4 (d, 1H); 3.16 (d, 1H)

### 2) Experimental method

### 2-1) Cell culture

Fibroblasts (CRL-2795; American Type Culture Collection, VA) were cultured in a Dulbecco's Modified Eagle Medium (Hyclone, Logan, UT), human uterine microvascular endothelial cells (PromoCell GmbH, Heidelberg, Germany) were cultured in an MV2 medium (PromoCell GmbH), and human hepatic stellate cells (Innoprot, Bizkia, Spain) were cultured in a P60126 medium (Innoprot).

All cells were maintained under a 5% humidified carbon dioxide condition at 37 °C. The cultured cells were exposed to each of PDGF, TGF_{β}, and the compounds of Formulas A1 to A9 (CBM-N1 ~ N9) for 24 hours, followed by performing experiments.

### 2-2) Construction of liver disease mouse models

To confirm the effects of the compounds of Formula A1 to A5 (CBM-N1 ~ N5) according to the present invention on liver inflammation and fibrosis, subsequent experiments were performed on C57BL/6 wild-type mouse (purchased from Orient Bio). First, to induce liver disease in mice, thioacetamide (TAA) were administered to the mice (Experiment A), or the mice were raised on a western diet inducing fatty liver disease (Experiment B). The mice were divided into a normal control, a disease-induced group, and a drug-administered group, and among these three groups, 15 to 100 mice were used in each group for Experiment A, and 10 mice were used in each group for Experiment B. A drug treatment method for the mice in each group is as follows:
(1) Normal control: The normal control in Experiment A was intraperitoneally injected three times a week with the same amount of TAA solvent used when TAA was injected into a disease-induced group, and the normal control in Experiment B was raised on a normal diet. In both of the Experiments A and B, a CBM-N1 solvent was injected using an oral tube at the same amount as that of CBM-N1 injection five times a week. The TAA solvent was distilled water, the CBM-N1 solvent and a derivative thereof were a 1:1 mixture of DMSO and distilled water. In the accompanying drawings, C or Control refers to a normal control.
(2) Disease-induced group: The disease-induced group in the Experiment A was intraperitoneally injected with TAA at 100 mg/kg three times a week, the disease-induced group in the Experiment B was raised on a western diet (WD, 45% saturated fat, 0.2% cholesterol, and water containing fructose and glucose). All CBM-N1 solvents in the Experiments A and B were administered using an oral tube at the same amount as that of CBM-N1 administration five times a week. In the accompanying drawings, TAA refers to a group in which a disease is induced by TAA, and WD refers to a group in which a disease is induced by a western diet.
(3) Drug-administered group: In the Experiment A, the compounds of Formulas 1 to 5 (50 mg/kg/day, 5 times/week) were administered with TAA (100 mg/kg, 3 times/week), and in the Experiment B, CBM-N1 to N5 (50 mg/kg/day, 5 times/week) were administered with a western diet. The mice treated by the above-described method for 16 weeks were instantly killed by excessively administering an anesthetic, and then the livers and blood were extracted. In the accompanying drawings, TAA+ CBM-N1, TAA+CBM-N2, TAA+CBM-N3, TAA+CBM-N4 and TAA+CBM-N5 refer to disease-induced groups to which the compounds of Formulas A1 to A5 were administered, respectively.

### 2-3) Construction of pulmonary inflammation and fibrosis mouse models

To confirm the effect of the compound of Formula A9 (CBM-N9) on pulmonary inflammation and fibrosis caused by bleomycin, the following experiments were performed on C57BL/6 wild-type mice. First, the mice were divided into a normal control, a disease-induced group, and a drug-administered group, and ten mice were included in each of the three groups. A drug treatment method for the mice in each group is as follows:
(1) Normal control: The same amount of distilled water as used when the bleomycin was applied in the disease-induced group was instilled intratracheally. In addition, a CBM-N9 solvent was intraperitoneally injected at the same amount used when CBM-N9 was administered to the disease-induced group to be described below five times a week.
(2) Disease-induced group: 1.5 units of bleomycin was instilled intratracheally. In addition, a CBM-N9 solvent was intraperitoneally injected at the same amount used in CBM-N9 administration five times a week.
(3) Drug-administered group: 1.5 units of bleomycin was intratracheally instilled. In addition, CBM-N9 (50 mg/kg) was injected intraperitoneally five times a week.

The mice treated with the drug for 4 weeks in the same manner as described above were instantly killed by excessively administering an anesthetic, and then the lungs were extracted.

### 2-4) Preparation of paraffin tissue samples of liver and lung tissues and observation of morphological changes thereof

To histologically confirm the therapeutic effect of the compound of Formula A1 (CBM-N1) or the compound of Formula A9 (CBM-N9) in each mouse model, a paraffin tissue sample was prepared. Liver and lung tissues were fixed with a paraformaldehyde solution, and sliced to a thickness of 1 to 2 mm. The sectioned tissues were embedded in paraffin, sliced to a thickness of 4 µm to remove paraffin with xylene, and the xylene was removed with ethanol, followed by washing with tap water. The resulting tissues were subjected to hematoxylin and eosin staining (H&E staining) or immunohistochemistry.
(1) H&E staining: Nuclei were first stained (blue) with a Harris hematoxylin staining solution for 5 minutes, and counter-stained (pink) with an eosin solution.
(2) Immunohistochemistry for inflammation markers: Inflammation markers (CD82 and CD45) were stained with specific antibodies, and lymphoid cells were stained brown.
(3) Immunohistochemistry for fibrosis markers: Collagen was stained by Masson's trichrome staining, or reticulin fibers were stained by reticulin staining.

### 2-5) Liver function test

Aspartic acid aminotransferase (AST, GOT) and alanine aminotransferase (ALT, GPT), total bilirubin and albumin concentrations were measured using blood collected from liver disease mouse models. A method for liver function testing is shown in Table 1 below.

**[Table 1]**

| Test item | Albumin | AST(SGOT) | ALT(SGPT) | Total bilirubin |
|---|---|---|---|---|
| Test method | Colorimetry (BCG method) | Modified IFCC UV (No pyridoxal phosphate and sample blank) | Modified IFCC UV (No pyridoxal phosphate and sample blank) | Colorimetry |
| Kit name/ manufacturer/ manufacturing country | ALB2/ Roche/ Germany | Aspartate aminotransferase for IFCC/Roche/Germany | Alanine aminotransferase for IFCC/Roche/Germany | Bilirubin total Gen.3/ Roche/Germany |
| Analyzer name/ manufacturer/ country | Cobas 8000c702/ Roche/ Germany | Cobas 8000c702/ Roche/ Germany | Cobas 8000c702/ Roche/ Germany | Cobas 8000c702/ Roche/ Germany |

### 2-6) Real-time polymerase chain reaction (Real-time PCR) analysis

The mRNA expression levels of inflammation or fibrosis factors in extracted liver tissue were measured by real-time PCR. RNA of the liver tissue was isolated with a TRIzol reagent (Molecular Research Center, Cincinnati, OH), and single-stranded cDNA was synthesized using BcaBEST polymerase (TakaraShuzo), followed by a polymerase chain reaction.

Primer sequences (SEQ ID NOs: 1 to 30) of inflammatory cytokines and fibrosis markers used herein are shown in Tables 2 to 4 below.

**[Table 2] Primer sequences for K_{ca}2.3 channel**

| Classification | Sense | SEQ. ID. NO: | Anti-sense | SEQ. ID. NO: |
|---|---|---|---|---|
| K_{Ca}2.3 | F- CCCGGCTCCTCTCCTGGCTT | 1 | R- GAAGTGGGGGCCCTGAACGC | 2 |
| mGAPDH | F-CCGTATTGGGCGCCTGG TCA | 3 | R- CCGGCCTTCTCCATGGTGGT | 4 |

**[Table 3] Primer sequences for inflammatory cytokines**

| Classification | Sense | SEQ. ID. NO: | Anti-sense | SEQ. ID. NO: |
|---|---|---|---|---|
| TNFα | F-CCCCAAAGGGATGAGAAGTT | 5 | R-CACTTGGTGGTTTGCTACGA | 6 |
| CCL2 | F-CCCCAAGAAGGAATGGGTCC | 7 | R-TGCTTGAGGTGTTTGTGGAA | 8 |
| TGF | F-TGGAGCAACATGTGGAACTC | 9 | R-TGCCGTACAACTCCAGTGAC | 10 |
| IL1α | F-GAGCCGGGTGACAGTATCAG | 11 | R-ACTTCTGCCTGACGAGCTTC | 12 |
| IL6 | F- ACCAGAGGAAATTTTCAATAGGC | 13 | R- TGATGCACTTGCAGAAAACA | 14 |
| MIP-2 | F-AGACAGAAGTCATAGCCA CTCTCAAG | 15 | R- CCTCCTTTCCAGGTCAGTTAGC | 16 |
| IL-12 | F-CAGTTGGCCAGGGTCATTC | 17 | R-GATGTCTTCAGCAGTGCAGG | 18 |
| mGAPDH | F-CCGTATTGGGCGCCTGGTCA | 19 | R-CCGGCCTTCTCCATGGTGGT | 20 |

**[Table 4] Primer sequences for fibrosis markers**

| Classification | Sense | SEQ. ID. NO: | Anti-sense | SEQ. ID. NO: |
|---|---|---|---|---|
| Col1α | F- ACAGTCCAGTTCTTCATTGC | 21 | R- GCACTCTTCTCCTGGTCCTG | 22 |
| Col3α | F-GCACAGCAGTCCAACGT AGA | 23 | R- TCTCCAAATGGGATCTCTGG | 24 |
| Col4α | F-AACAACGTCTGCAACTT CGC | 25 | R- ACCGCACACCTGCTAATGAA | 26 |
| TGFR1 | F-AGCTCCTCATCGTGTTGG TG | 27 | R- TGCAGTGGTCCTGATTGCAG | 28 |
| TGFR2 | F-ACGTTCCCAAGTCGGATG TG | 29 | R- TGCAGTGGTCCTGATTGCAG | 30 |
| α-SMA | F-CTGACAGAGGCACCACTG AA | 31 | R- CATCTCCAGAGTCCAGCACA | 32 |
| mGAPDH | F-CCGTATTGGGCGCCTGG TCA | 33 | R- CCGGCCTTCTCCATGGTGGT | 34 |

### 2-7) Western blotting analysis

Cells were lysed with a protein extraction buffer solution, a protein concentration in a supernatant was determined by Bradford analysis, and 30 µg of the protein was loaded on an SDS-PAGE gel and then transferred to a nitrocellulose membrane. The nitrocellulose membrane was blocked with 5% BSA-containing TBST (10 mM Tris-HCl, 150 mM NaCl, and 0.1%(v/v) Tween-20, pH 7.6) at room temperature for 1 hour. Blots were incubated overnight with primary antibodies, and then incubated with horseradish peroxidase-conjugated secondary antibodies for 1 hour. Bands were visualized by chemiluminescence. Data collection and processing were performed using an image analyzer (LAS-3000) and IMAGE GAUSE software (Fuji film, Japan).

### 2-8) Test method for MTT cell proliferation

Cells were seeded in 96-well plates at 2×10⁴ cells/well, and then exposed to TGF_{β}, which promotes cell proliferation, for 24 hours. In addition, 0.1 mg of MTT was added to each well, followed by exposure for 4 hours at 37 °C. Afterward, the culture medium was removed, the cells were lysed with dimethyl sulfoxide, and then absorbance was measured at 590 nm using the following devices.

### 2-9) Electrophysiological analysis

A whole cell current through a cell membrane in the isolated and cultured single cells was measured using a patch-clamp technique. A voltage ramp was applied from -100 mV to +100 mV using a micro glass electrode in whole-cell voltage clamped cells, and the resulting current was amplified using an amplifier (EPC-10, HEKA, Lambrecht, Germany), followed by recording at a sampling rate of 1 to 4 kHz.

A standard external solution contained 150 mM NaCl, 6 mM KCl, 1.5 mM CaCl₂, 1 mM MgCl₂, 10 mM HEPES and 10 mM glucose at pH 7.4 (titrated with NaOH), and a micro glass electrode (pipette) solution contained 40 mM KCl, 100 mM K-aspartate, 2 mM MgCl₂, 0.1 mM EGTA, 4 mM Na₂ATP and 10 mM HEPES at pH 7.2 (titrated with KOH). A free Ca²⁺ concentration in the pipette solution was adjusted to 1 µM by adding an appropriate amount of Ca²⁺ in the presence of 5 mM EGTA (calculated with CaBuf; G. Droogmans, Leuven, Belgium).

The K_{Ca}2.3 current was separated by the following method. Among the currents recorded by injecting 1 µM Ca²⁺ into whole-cell voltage clamped cells using a glass electrode and applying 1-ethyl-2-benzimidazolinone (1-EBIO, 100 µM) activating the K_{Ca}2.3 current, a current inhibited by apamin (200 nM), which is a K_{Ca}2.3 channel inhibitor, was determined as the K_{Ca}2.3 current, and the recorded current was divided by cell capacitance and normalized.

### 2-10) Statistical analysis

Experimental results were expressed as mean ± standard deviation (S.E.M). Statistical analysis was performed using a Student's t-test, and P≤0.05 was determined as significant difference.

### 3) Results of experiments using cultured cells

The experiments were performed to identify effects of the compounds of Formulas A1 to A9 (CBM-N1~N9) according to the present invention on *in vitro* K_{Ca}2.3 channel expression, and whether the compounds of Formulas A1 to A9 (CBM-N1~N9) inhibit fibrosis.

### 3-1) Effects of growth factors and CBM-N1 on K_{Ca}2.1 and K_{Ca}3.1 channels

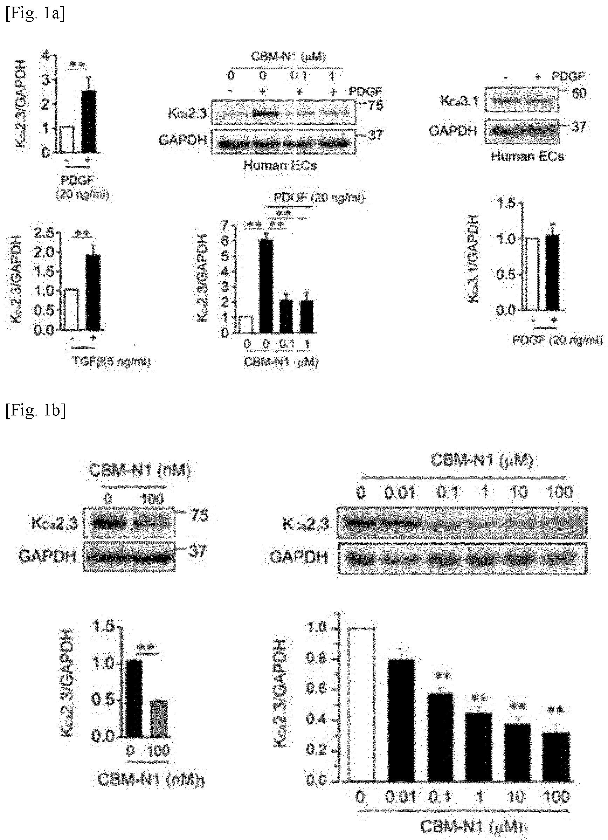

FIG. 1A shows the effect of each of PDGF, TGF_{β}, and CBM-N1 on the expression of a K_{Ca}2.3 channel or K_{Ca}3.1 channel in vascular endothelial cells. When the vascular endothelial cells were exposed to PDGF (20 ng/ml) or TGF_{β} (5 ng/ml) for 24 hours, the expression of the mRNA (left panel) and protein (middle panel) of the K_{Ca}2.3 channel increased. On the other hand, the expression of the K_{Ca}3.1 channel did not increase due to PDGF treatment (right panel). In addition, when cells in which the expression of the K_{Ca}2.3 channel protein was increased with PDGF were treated with CBM-N1 for 24 hours, the expression of the K_{Ca}2.3 channel protein significantly decreased (middle panel).

FIG. 1B shows the effect of CBM-N1 on the expression of a K_{Ca}2.3 channel protein in fibroblasts (left panel) or hepatic stellate cells (right panel). As a result, the expression level of the stable K_{Ca}2.3 channel was reduced in fibroblasts by CBM-N1 treatment, and reduced in hepatic stellate cells in a concentration-dependent manner.

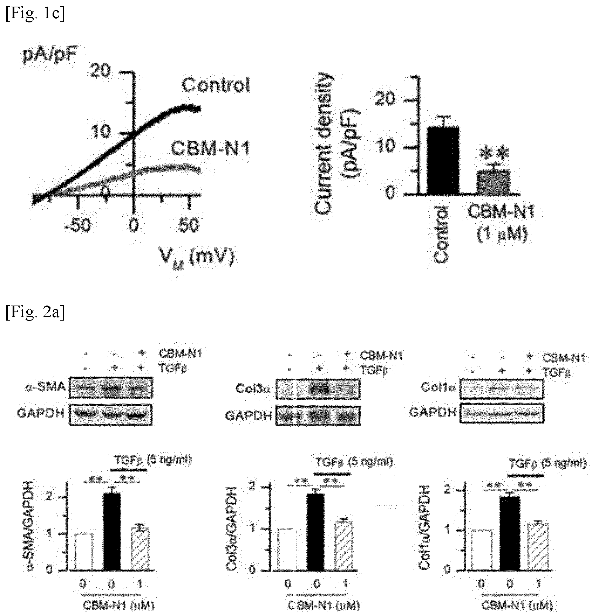

FIG. 1C shows the effect of the inhibition of K_{Ca}2.3 channel expression in hepatic stellate cells by CBM-N1 on K_{Ca}2.3 current. K_{Ca}2.3 current density at a membrane potential of +50 mV were compared between cells exposed to CBM-N1 for 24 hours and cells not exposed to CBM-N1. The K_{Ca}2.3 current densities were 18.98±4.17 pA/pF in the cells not exposed to CBM-N1 and 7.77±2.65 mV/pF in the cells exposed to CBM-N1. That is, the K_{Ca}2.3 current was significantly reduced by the inhibition of K_{Ca}2.3 channel expression due to CBM-N1. As described above, the decreased K_{Ca}2.3 current in the cells in which the expression of the K_{Ca}2.3 channel protein was reduced by CBM-N1 means a decrease in cell membrane expression of the channel protein by CBM-N1.

### 3-2) Inhibitory effect of CBM-N1 on fibrosis

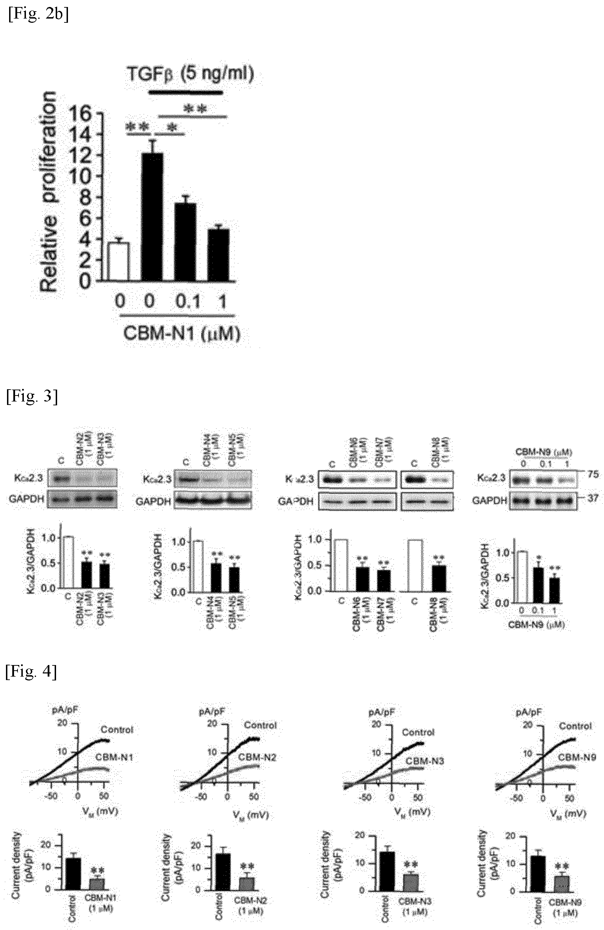

FIG. 2 shows the inhibitory effect of CBM-N1 on TGF_{β}-induced fibrosis in fibroblasts. The fibrosis-inducing effect by TGF_{β} was determined by expression levels of fibrosis markers (FIG. 2A) and a cell proliferation-inducing effect (FIG. 2B). When the fibroblasts were exposed to TGF_{β} (5 ng/ml) for 24 hours, the amounts of the fibrosis markers such as α-smooth muscle actin (α-SMA), collagen 1α (Col1α) and collagen 3α (Col3α) proteins increased, and cell proliferation was promoted. When the fibroblasts were exposed to TGF_{β} +CBM-N1 for 24 hours, the expression levels of the fibrosis markers were reduced, and cell proliferation was inhibited. These results show that CBM-N1 inhibits fibrosis induced by a fibrosis-inducing factor.

### 3-3) Effect of CBM-N1 and its derivatives (CBM-N2 to CBM-N9) on K_{Ca}2.3 channel expression and cell proliferation

FIG. 3 shows the effect of CBM-N1 derivatives on the expression of a K_{Ca}2.3 channel protein in hepatic stellate cells. Specifically, by the exposure of the cells to CBM-N2, CBM-N5, CBM-N8 and CBM-N9 for 24 hours, the expression of the K_{Ca}2.3 channel was significantly reduced.

FIG. 4 shows the effect of the inhibition of K_{Ca}2.3 channel expression by CBM-N2 to CBM-N4, and CBM-N9 in hepatic stellate cells on K_{Ca}2.3 current. As a result of the comparison of K_{Ca}2.3 current densities at a membrane potential of +50 mV between the cells in which the K_{Ca}2.3 channel expression is inhibited by exposure to the compound for 24 hours and the cells not exposed to the compound, due to the exposure to CBM-N2 to CBM-N4 and CBM-N9, the K_{Ca}2.3 current densities were significantly decreased in cells in which the K_{Ca}2.3 channel expression was reduced.

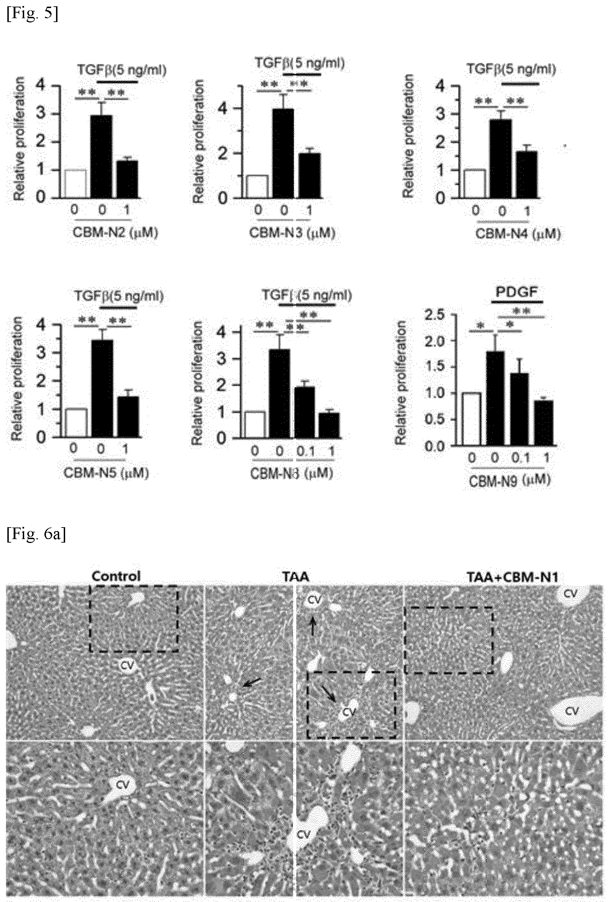

FIG. 5 shows the effect of CBM-N2 to CBM-N5, CBM-N8 and CBM-N9 on TGF_{β} or PDGF-induced cell proliferation in fibroblasts. When the cells were exposed to a fibrosis-inducing factor such as TGF_{β} (5 ng/ml) or PDGF (20 ng/ml) for 24 hours, the proliferation of fibroblasts significantly increased, and the cell proliferation was significantly reduced by the CBM-N2 to CBM-N5, CBM-N8 and CBM-N9.

### 4) Results from liver disease mouse models

To identify the therapeutic effects of the compounds of Formulas A1 to A5 (CBM-N1 to CBM-N5) in liver disease mouse models, these experiments were performed.

### 4-1) Histological or immunohistological analysis

FIG. 6A shows H&E staining results for the liver tissues, and the part represented by a dotted line in the upper panel was enlarged and shown in the lower panel. In a disease-induced group (TAA), TAA-induced inflammation occurs in a region near the central vein (CV), which can be confirmed by inflammation cells having large nuclei concentrated near the CV (arrows in FIG. 6A). Particularly, compared with the normal control, in the disease-induced group, inflammation cells significantly increased, and in the drug-administered group (TAA+CBM-N1), compared with the disease-induced group, the number of inflammation cells significantly decreased.

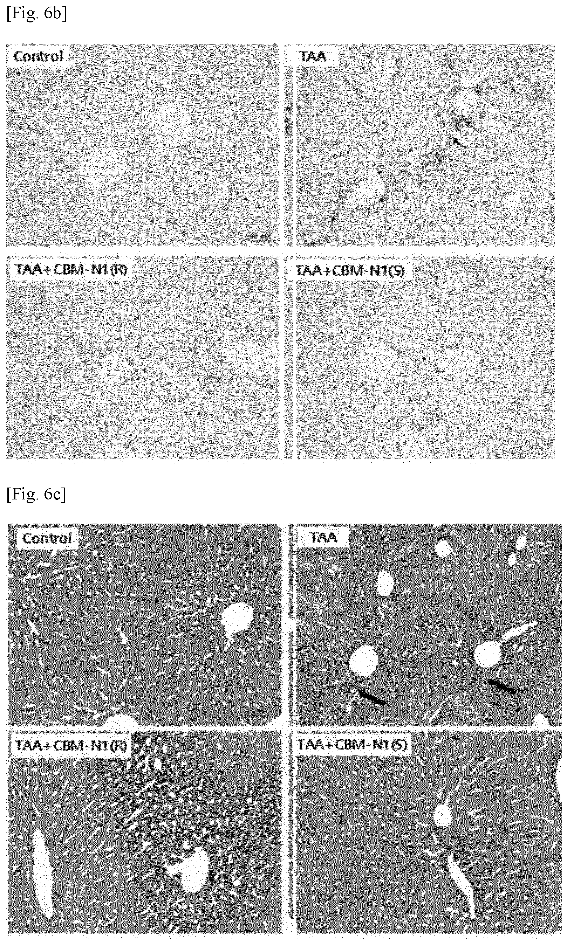

FIG. 6B shows staining results of an inflammation marker, CD82, in the liver tissue, in which no cells stained brown were observed in liver tissue of the normal control (Control), indicating that there were no lymphoid cells. On the other hand, in the liver tissue of the group in which a disease is induced by TAA (TAA), many cells stained brown were found between CVs. However, a very small number of cells stained brown were found in the liver tissue (TAA+CBM-N1(R) or TAA+CBM-N1(S)) in a drug-administered group treated with TAA and a CBM-N1 R-isomer or S-isomer.

FIG. 6C shows results of Masson's trichrome staining of collagen fibers in the liver tissues, and the collagen was stained blue. The liver tissue in the normal control (Control) is a healthy state in which fibrosis has not progressed yet, whereas the liver tissue in the disease-induced group (TAA) is stained blue (indicated with arrows) near CVs or between CVs, demonstrating the progression of fibrosis. However, in the liver tissue in the drug-administered group (TAA+CBM-N1(R), TAA+CBM-N1(S)) to which TAA and a CBM-N1 R-isomer or S-isomer were administered, fibrosis was very slightly observed around CVs.

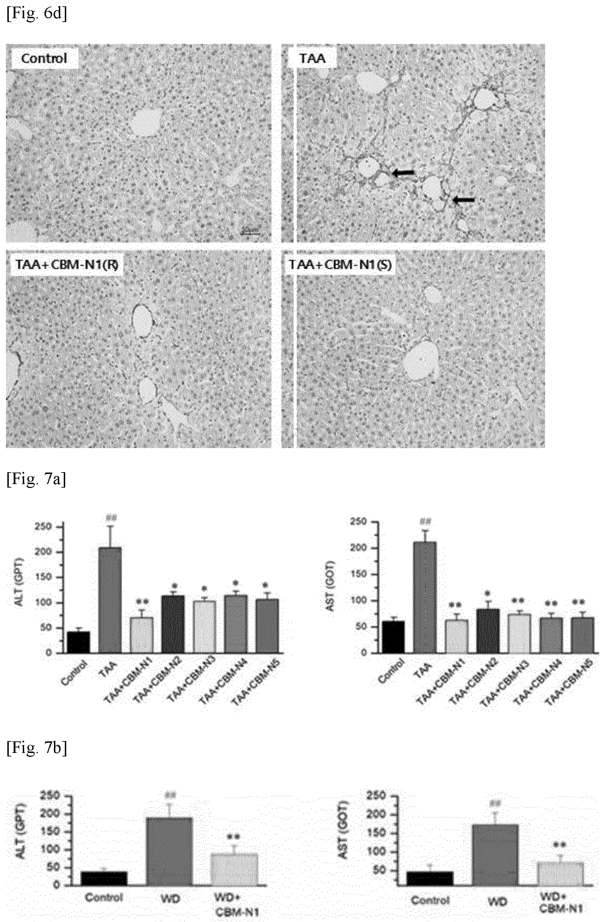

FIG. 6D shows staining results for reticulin fibers in the liver tissues, and the reticulin fibers were stained black. No reticulin fiber was observed in the liver tissue from the normal control (Control), whereas in the liver tissue in the disease-induced group (TAA), reticulin fibers (indicated with arrows) were observed around CVs and between CVs. In addition, in the liver tissue (TAA+CBM-N1(R) or (TAA+CBM-N1(S)) in the drug-administered group administered with TAA and a CBM-N1 R-isomer or S-isomer, the reticulin fibers were very slightly observed only aroundr CVs.

### 4-2) Liver function test through blood ALT and AST analyses

FIG. 7A shows results of liver function testing for a normal control, a TAA-mediated disease-induced group, and drug-administered groups treated with CBM-N1 to CBM-N5. In a normal control (Control), a disease-induced group (TAA), and drug-administered groups (TAA+CBM-N1, TAA+CBM-N2, TAA+CBM-N3, TAA+CBM-N4, and TAA+CBM-N5), ALT levels were 41.6±7.9 units/L, 209.0±42.4 units/L, 70.3±14.7 units/L, 113.4±7.9 units/L, 103.0± 6.9 units/L, 114.1±8.8 units/L and 106.4±12.8 units/L, respectively, and AST blood levels were 60.4±7.5 units/L, 211.1±22.4 units/L, 62.7±11.6 units/L, 83.6±14.8 units/L, 73.4±7.4 units/L, 66.6±9.4 units/L, and 67.7±10.2 units/L, respectively.

In addition, FIG. 7B shows a test result of a disease-induced group by western diet, and in a normal control (Control), a group in which a disease was induced by a western diet (WD), and a drug-administered group (WD+CBM-N1), ALT levels were 38.7±9.7 units/L, 189.8±37.6 units/L and 87.2±24.7 units/L, and AST blood levels were 47.4±18.2 units/L, 173.5±31.5 units/L and 71.4±19.8 units/L, respectively.

From the test results, it can be seen that liver dysfunction induced by TAA or a western diet is significantly recovered by compounds of Formulas A1 to A5 (50 mg/kg/day).

### 4-3) Real time PCR for inflammation markers

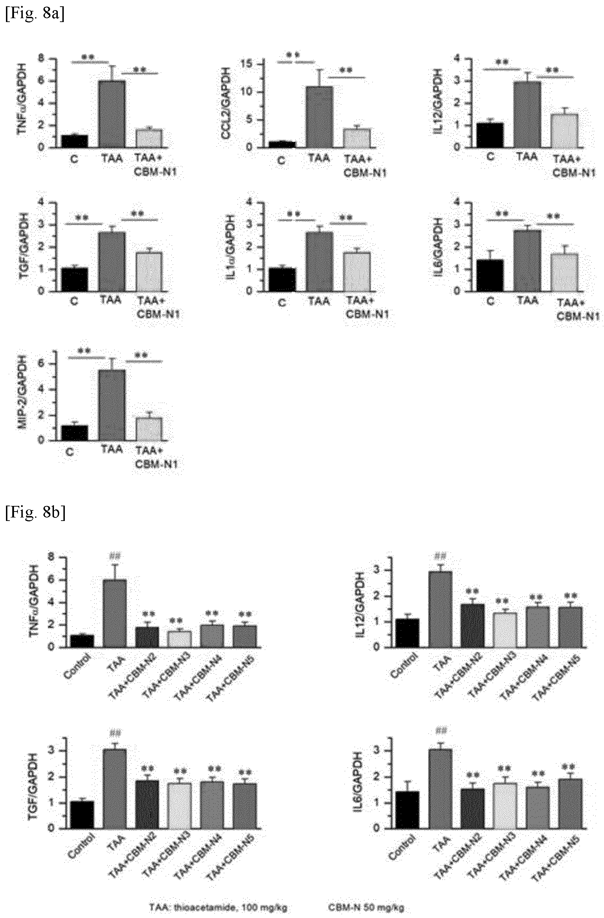

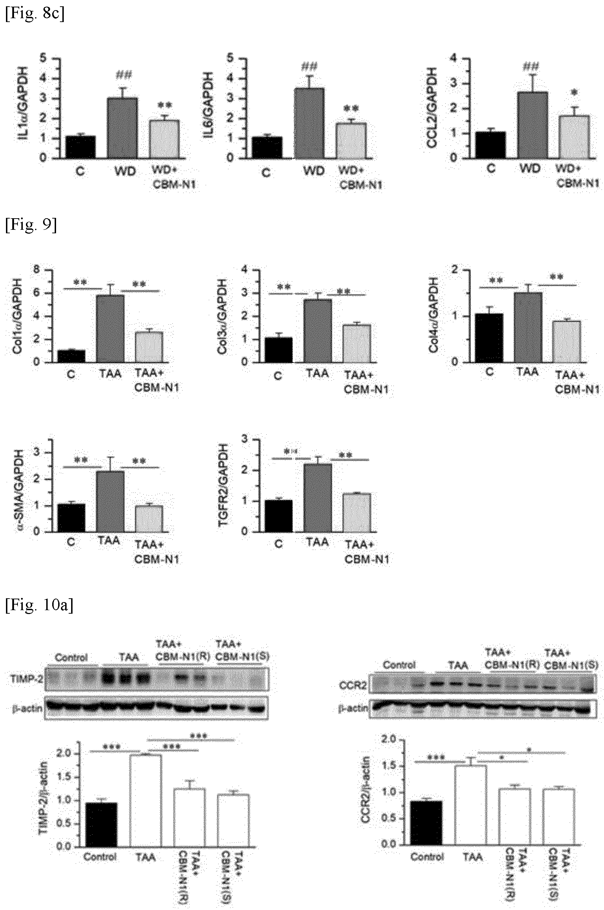

FIG. 8 shows results of comparing the mRNA expression of inflammatory cytokines in a normal control, a disease-induced group and a drug-administered group. As the inflammation markers, tumor necrosis factor alpha (TNFα), chemoattractant protein-1 (CCL2), interleukin-12 (IL12), transforming growth factor (TGF), IL1α, IL6, and macrophage inflammatory protein-2 (MIP-2), which increase when inflammation occurs, were used. The mRNA level of an inflammation factor increased in the disease-induced group (TAA) as compared with the normal control (Control), and decreased in the CBM-N1-administered group (TAA+CBM-N1) as compared with the disease-induced group (TAA) (FIG. 8A).

The mRNA level of an inflammation factor also decreased in the CBM-N2 to CBM-N5-administered groups (TAA+CBM-N2, TAA+CBM-N3, TAA+CBM- N4, and TAA+CBM-N5) compared with the disease-induced group (FIG. 8B). Therefore, it can be seen that the compounds of Formulas A1 to A5 decreased the expression of an inflammatory cytokine, thereby having a therapeutic effect on an inflammatory liver disease caused by TAA.

In addition, FIG. 8C shows results of comparing the mRNA expressions of inflammatory cytokines in a normal control (Control), a group in which a disease was induced by a western diet (WD), and a drug-administered group (WD+CBM-N1). Here, as inflammatory cytokines, CCL2, IL6 and IL1α were measured. The mRNA expression levels of these inflammation factors increased in the disease-induced group as compared with the normal control, and decreased in the drug-administered group as compared with the disease-induced group.

### 4-4) Real time PCR for fibrosis markers

FIG. 9 shows results of comparing mRNA expression levels of fibrosis markers in a normal control, a disease-induced group and a drug-administered group. As the fibrosis markers, Col1α, Col3α, Col4α, α-SMA and transforming growth factor receptor 2 (TGFR2) were used. The fibrosis markers increased in the disease-induced group (TAA) compared with the normal control (Control), indicating that fibrosis progresses due to inflammation. However, it was confirmed that, in the CBM-N1-administered group (TAA+CBM-N1), the levels of these fibrosis factors decreased.

### 4-5) Effect on protein expression of inflammation marker or fibrosis marker proteins

FIG. 10A shows the effects of the R-isomer and S-isomer of CBM-N1 on the protein expression of inflammation markers in TAA-mediated liver disease mouse models. Compared with the control (Control), the protein expression levels of TIMP-2 and CCR2 greatly increased in liver tissue of the disease-induced group (TAA), indicating the progression of inflammation. However, in the CBM-N1 R-isomer or S-isomer-administered group [TAA+CBM-N1(R), TAA+CBM-N1(S)], protein expression levels of TIMP-2 and CCR2 decreased, confirming that inflammation was inhibited.

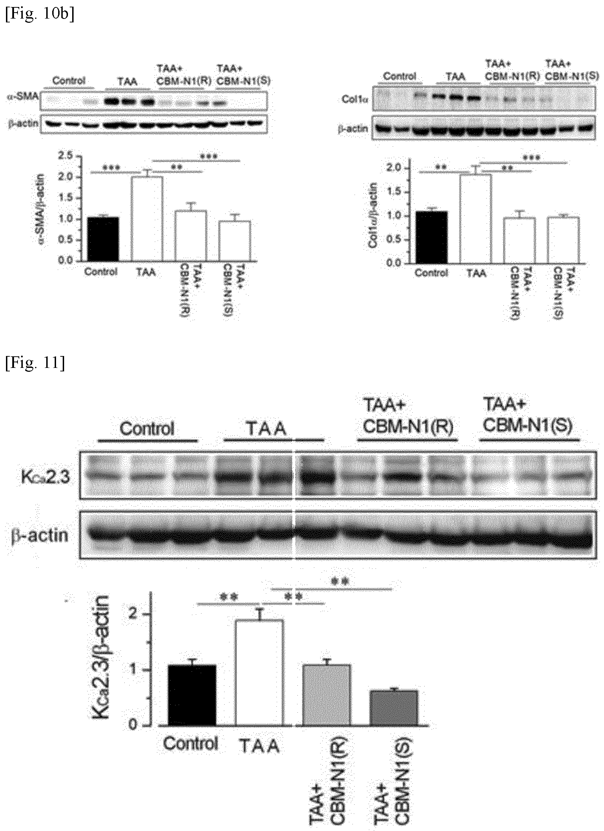

FIG. 10B shows the effect of the R-isomer or S-isomer of CBM-N1 on the expression of fibrosis marker proteins in TAA-mediated liver disease mouse models. Compared with the normal control (Control), protein expression levels of α-SMA and Col1α greatly increased in liver tissue of the disease-induced group (TAA), indicating the progression of fibrosis. However, the protein expression levels of α-SMA and Col1α greatly decreased in the CBM-N1 R-isomer or S-isomer-administered group, confirming that fibrosis was inhibited.

### 4-6) Effect on expression of K_{Ca}2.3 channel protein

FIG. 11 shows the effect of the R-isomer or S-isomer of CBM-N1 on the expression of a K_{Ca}2.3 channel protein in TAA-mediated liver disease mouse models. Compared with the normal control (Control), the expression level of the K_{Ca}2.3 channel protein greatly increased in liver tissue of the disease-induced group (TAA). However, the protein expression level of the K_{Ca}2.3 channel greatly decreased in the CBM-N1 R-isomer or S-isomer-administered group. These results show that a TAA-induced liver disease is associated with the increase in K_{Ca}2.3 expression, and the therapeutic effect of CBM-N1 is associated with the decrease in K_{Ca}2.3 expression.

### 5) Experimental result for CBM-N9 in lung disease mouse models

To identify the therapeutic effect of the compound of Formula A9 (CBM-N9) of the present invention in bleomycin-induced lung disease mouse models, this experiment was performed.

### 5-1) Histological or immunohistological analysis

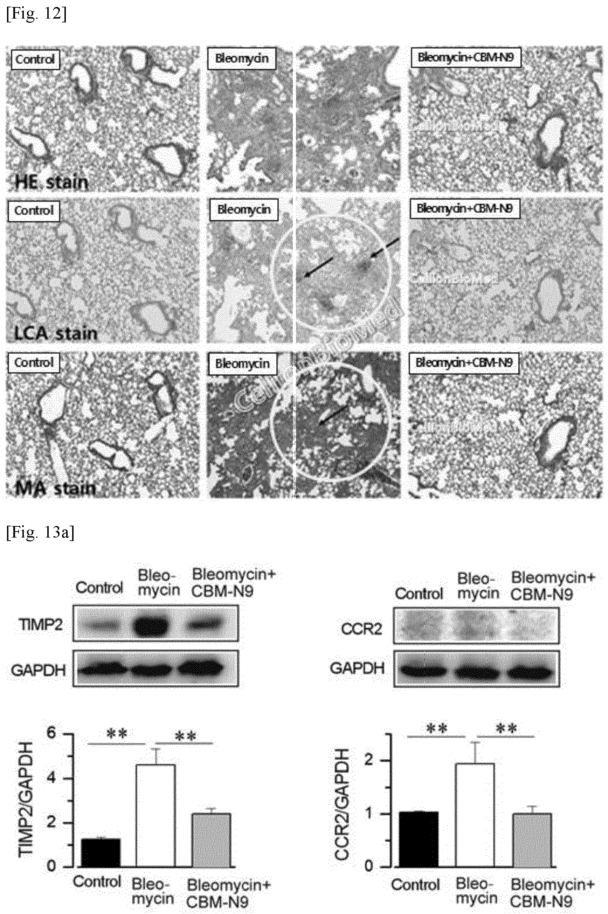

FIG. 12 shows results of H&E staining in lung tissue, immunohistochemistry for CD45 (leukocyte common antigen, LCA staining), and Masson's trichrome staining for collagen. It was confirmed that degrees of inflammation and fibrosis increased in the disease-induced group (Bleomycin) as compared to the normal control, and decreased in the CBM-N9-administered group (Bleomycin+CBM-N9) as compared with the disease-induced group.

### 5-2) Analysis of protein expression of inflammation or fibrosis markers

FIG. 13A shows the effect of CBM-N9 on the expression of inflammation markers in lung disease mouse models. Protein expression levels of inflammation markers such as TIMP-2 and CCR2 in lung tissue greatly increased in a disease-induced group (bleomycin) as compared with a normal control (Control), resulting in the progression of inflammation. The protein expression levels of TIMP-2 and CCR2 greatly decreased in a CBM-N9 drug-administered group (bleomycin+CBM-N9), confirming the inhibition of inflammation.

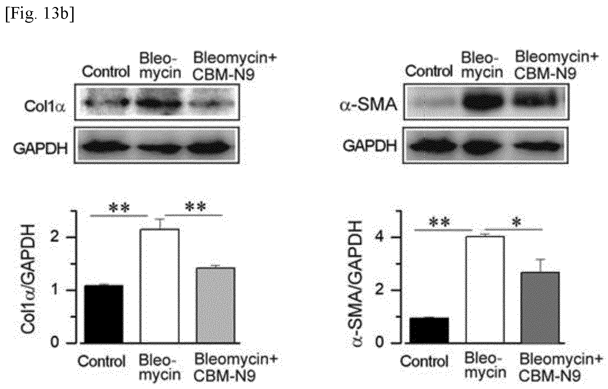

FIG. 13B shows the effect of CBM-N9 on the expression of fibrosis marker proteins in lung disease mouse models. The protein expression levels of fibrosis markers such as α-SMA and Col1α greatly increased in lung tissue of a disease-induced group (bleomycin) compared with a normal control (Control), indicating the progression of pulmonary fibrosis. On the other hand, the protein expression levels of TIMP-2 and CCR2 greatly decreased in a drug-administered group (bleomycin+CBM-N9), confirming the inhibition of fibrosis.

### 6) Evaluation and conclusion

As seen above, when culture cells were administered with the compounds of Formulas A1 to A9 according to the present invention *in vitro* for a long time (24 hours or 16 weeks), the effect of inhibiting fibrosis was exhibited by the decrease in expression of a K_{Ca}2.3 channel protein. Specifically, it was confirmed that, when cultured hepatic stellate cells, fibroblasts, and vascular endothelial cells were exposed to the compounds of Formulas A1 to A9 for 24 hours, the cell membrane expression of a K_{Ca}2.3 channel was inhibited, and the expression of fibrosis-related factors (α-SMA, Col1α, etc.) and cell proliferation by growth factors inducing fibrosis were inhibited.

In addition, it was confirmed that the compounds of Formulas A1 to A9 according to the present invention have inhibitory effects on inflammation and fibrosis in a liver disease-induced group even in an *in vivo* experiment for mouse models. Specifically, as a result of administering the compounds of Formulas A1 to A9 to liver disease or lung disease mouse models for 16 weeks, inflammation and fibrosis were significantly inhibited.

Meanwhile, as disclosed in Korean Patent Nos. 10-1345860 and 10-1414831 and U.S. Patent No. 9,259,412 corresponding thereto, the compounds of Formulas A1 to A5 of the present invention have effects of inhibiting the activity of a K_{Ca}3.1 channel due to K_{Ca}3.1 channel phosphorylation induced by cAMP. However, it is considered that the suppression of the activity of the K_{Ca}3.1 channel by increased cAMP will have little effect on fibrosis treatment.

The present invention relates to an effect exhibited when being exposed to the compounds of Formulas A1 to A5 for a short time (within several minutes), and the increased cAMP due to these compounds reached the highest level in approximately 20 minutes and then dramatically decreased such that the cAMP level became similar to that before drug administration within three hours (Endocrinology 144(4):1292-1300). Therefore, this is because the effect caused by cAMP is exhibited only for a short time, for example, at most, approximately one hour, and as in the case of the present invention, is unlikely to last for 24 hours or 16 weeks.

In addition, as confirmed in FIG. 1A of the present invention, when exposed to a fibrosis-inducing factor, PDGF, for 24 hours, K_{Ca}2.3 channel expression greatly increased, whereas K_{Ca}3.1 channel expression did not increase. According to this result, it may be concluded that, in a fibrotic process, an increase in K_{Ca}2.3 channel expression is a very important requisite, and the fibrosis suppressing effect of the compounds of Formulas A1 to A9 according to the present invention results from a decrease in K_{Ca}2.3 channel expression.

Meanwhile, in the present disclosure, due to realistic limitations, the above-described experiments for all of compounds belonging to the compounds of Formula A were not performed. However, in consideration of chemical activities of the compounds of Formula A and metabolic mechanisms *in vivo,* it is inferred that all of the compounds of Formula A have pharmacological effects the same as or similar to the compounds of Formulas A1 to A9.

## Claims

1. A composition for use in treating liver fibrosis or pulmonary fibrosis, comprising a benzhydryl thioacetamide compound selected from the group consisting of 2-(benzhydrylsulfinyl)acetamide, 2-(benzhydrylthio)-N-[(tetrahydrofuran-2-yl)methyl]acetamide, 2-(benzhydrylthio)-N-phenylacetamide, 2-(benzhydrylsulfinyl)-N-methylacetamide, 2-(benzhydrylsulfinyl)-N-[(tetrahydrofuran-2-yl)methyl]acetamide, 2-(benzhydrylthio)-ene-methylacetamide, 2-[bis(2-fluorophenyl)methanesulfinyl]acetamide, 2-[bis(3-fluorophenyl) methanesulfinyl]acetamide, and 2-[bis(4-fluorophenyl)methanesulfinyl]acetamide or a pharmaceutically acceptable salt thereof as an active ingredient.

2. The composition for use according to claim 1, wherein the compound is 2-(benzhydrylsulfinyl)acetamide (modafinil).

3. The composition for use according to claim 1, wherein the compound is 2-[bis(4-fluorophenyl)methanesulfinyl]acetamide (lauflumide).

4. The composition for use according to claim 1, wherein the compound has an effect of suppressing the expression of a KCa2.3 channel protein in a cell membrane.

## Patentansprüche

1. Zusammensetzung zur Behandlung von Leberfibrose oder Lungenfibrose, umfassend eine Benzhydrylthioacetamid-Verbindung, ausgewählt aus der Gruppe bestehend aus 2-(Benzhydrylsulfinyl)acetamid, 2-(Benzhydrylthio)-N-[(tetrahydrofuran-2-yl)methyl]acetamid, 2-(Benzhydrylthio)-N-phenylacetamid, 2-(Benzhydrylsulfinyl)-N-methylacetamid, 2-(Benzhydrylsulfinyl)-N-[(tetrahydrofuran-2-yl)}methyl]acetamid, 2-(Benzhydrylthio)-en-methylacetamid, 2-[Bis(2-fluorophenyl)methansulfinyl]acetamid, 2-[Bis(3-fluorophenyl)methansulfinyl]acetamid und 2-[Bis(4-fluorophenyl)methansulfinyl]acetamid oder ein pharmazeutisch akzeptables Salz davon als Wirkstoff.

2. Zusammensetzung zur Verwendung nach Anspruch 1, wobei die Verbindung 2-(Benzhydrylsulfinyl)acetamid (Modafinil) ist.

3. Zusammensetzung zur Verwendung nach Anspruch 1, wobei die Verbindung 2-[Bis(4-fluorophenyl)methansulfinyl]acetamid (Lauflumid) ist.

4. Zusammensetzung zur Verwendung nach Anspruch 1, wobei die Verbindung eine Wirkung des Unterdrückens der Expression eines KCa2.3-Kanalproteins in einer Zellmembran aufweist.

## Revendications

1. Composition destinée au traitement de la fibrose hépatique ou pulmonaire, comprenant un composé de benzhydryl thioacétamide choisi parmi le groupe constitué du 2-(benzhydrylsulfinyl)acétamide, 2-(benzhydrylthio)-N-[(tétrahydrofuran-2-yl)méthyl]acétamide, 2-(benzhydrylthio)-N-phénylacétamide, 2-(benzhydrylsulfinyl)-N-méthylacétamide, 2-(benzhydrylsulfinyl)-N-[(tétrahydrofuran-2-yl)méthyl]acétamide, 2-(benzhydrylthio)-ène-méthylacétamide, 2-[bis(2-fluorophényl)méthanesulfinyl]acétamide, 2-[bis(3-fluorophényl)méthanesulfinyl]acétamide, et 2-[bis(4-fluorophényl)méthanesulfinyl]acétamide ou un sel pharmaceutiquement acceptable de celui-ci comme ingrédient actif.

2. Composition à utiliser selon la revendication 1, dans laquelle le composé est le 2-(benzhydrylsulfinyl)acétamide (modafinil).

3. Composition à utiliser selon la revendication 1, dans laquelle le composé est le 2-[bis(4-fluorophényl)méthanesulfinyl]acétamide (lauflumide).

4. Composition à utiliser selon la revendication 1, dans laquelle le composé a pour effet de supprimer l'expression d'une protéine de canal KCa2.3 dans une membrane cellulaire.
